(19) Europäisches Patentamt
European Patent Office
Office européen des brevets

(11) EP 1 619 200 A1

# (12) EUROPEAN PATENT APPLICATION

(43) Date of publication:
**25.01.2006 Bulletin 2006/04**

(51) Int Cl.:
***C07J 73/00*** *(2006.01)*

(21) Application number: **04015746.3**

(22) Date of filing: **05.07.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(71) Applicant: **Lonza AG**
**4052 Basel (CH)**

(72) Inventors:
- **Bessard, Yves**
**3960 Sierre (CH)**
- **Kuo, David L.**
**Short Hills,**
**New Jersey 07078 (US)**

## (54) Process for the preparation of 4-azasteroids

(57) The invention relates to a process for the preparation of 4-steroids of the formula

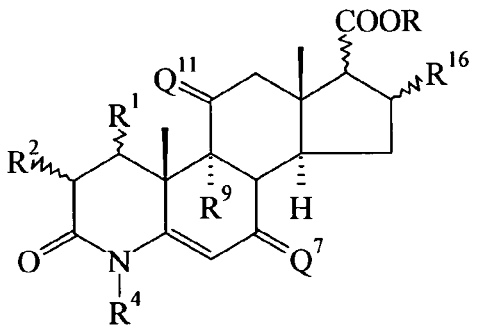

I,

$R^1$ and $R^2$ are independently selected from the group consisting of hydrogen, F, Cl, Br, I, $C_{1-6}$-alkyl and $C_{1-6}$-alkoxy, and

$R^4$ is selected from the group consisting of hydrogen, (*N*,*N*-di-$C_{1-6}$-alkylamino)methyl, 2-(*N*,*N*-di-$C_{1-6}$-alkylamino)ethyl, $C_{1-6}$-alkyl, $C_{1-6}$-alkoxy, phenyl and benzyl, and

$Q^7$ represents a carbonyl oxygen atom or is $R^{7-1}$ and $R^{7-2}$, wherein one of $R^{7-1}$ and $R^{7-2}$ is hydrogen and the other is selected from the group consisting of hydrogen, F, Cl, Br, I, $C_{1-6}$-alkyl and $C_{1-6}$-alkoxy, and

$R^9$ represents hydrogen or F, and

$Q^{11}$ represents a carbonyl oxygen atom or is $R^{11-1}$ and $R^{11-2}$, wherein one of $R^{11-1}$ and $R^{11-2}$ is hydrogen and the other is selected from the group consisting of hydrogen, cyano, cyano-$C_{1-3}$-alkyl, acetoxy, COOH and $COO^-M^+$, wherein $M^+$ is $Na^+$, $K^+$ or $NH_4^+$, and

$R^{16}$ is selected from the group consisting of hydrogen, cyano, $C_{1-3}$-alkyl, cyano-$C_{1-3}$-alkyl, acetoxy, COOH and $COO^-M^+$, wherein $M^+$ is $Na^+$, $K^+$ or $NH_4^+$, and

COOR represents COOH or $COO^-M^+$, wherein $M^+$ is $Na^+$, $K^+$ or $NH_4^+$.

The three step process comprises (i) a haloform reaction of a 17-acetyl steroid converting the acetyl group into a -COOR group, (ii) subsequent ozonolysis of the A-ring and (iii) reclosure of the A-ring by reaction with an appropriate nitrogen compound of formula $H_2NR^4$ to afford a compound of formula I above.

EP 1 619 200 A1

## Description

The invention relates to a process for the preparation of 4-azasteroids of the formula

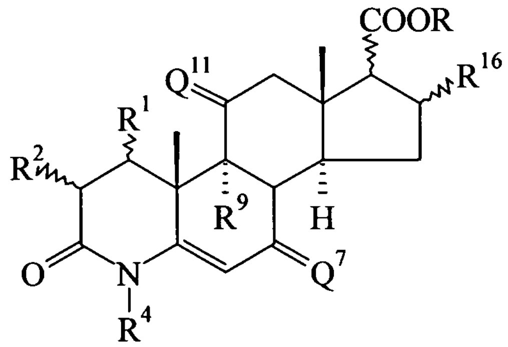

wherein $R^1$, $R^2$, $R^4$, $Q^7$, $R^9$, $Q^{11}$, $R^{16}$ and COOR are as defined below,
The three step process comprises (i) a haloform reaction of a 17-acetyl steroid converting the acetyl group into a -COOR group, (ii) subsequent ozonolysis of the A-ring and (iii) reclosure of the A-ring by reaction with an appropriate nitrogen compound of formula $H_2NR^4$ to afford a compound of formula I.

Compounds of formula I are key intermediates for the production of pharmaceutically active 4-azasteroids, e.g. 17-substituted-4-aza-5α,17β-androsten-3-ones, among them Finasteride (Proscar®), showing powerful 5α-reductase inhibition useful in the treatment of acne, alopecia and benign prostatic hypertrophy.

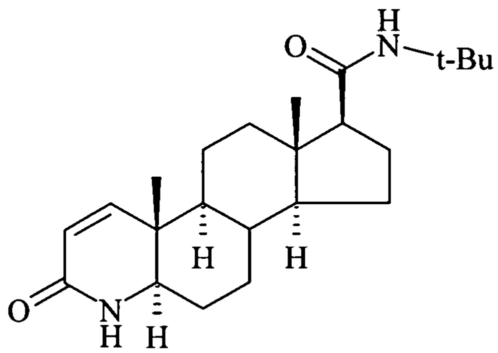

Finasteride (Proscar®)

A detailed investigation on the inhibition activity of 5α-reductase by several 4-azasteroids and their preparation is disclosed in Rasmusson, Gary H. et al. *J. Med. Chem.* 29**, 1986,** 2298-2315.

Rasmusson, Gary H. et al. obtained 17β-carboxy-5-oxo-A-nor-3,5-secoandrostan-3-oic acid in about 75% yield by oxidation of 3-oxo-4-androstene-17β-carboxylic acid into using expensive oxidation agents such as $NaIO_4$ and $KMnO_4$.

WO 90/15045 provides a process wherein the A-ring and an unsaturated acyl side chain of a steroid comprising are ozonolyzed simultaneously. The disadvantage of WO 90/15045 is the removal of the unsaturated acyl side chain, which leads to a substantial material loss. In the examples 1 to 3 four carbon and three oxygen atoms or eight carbon atoms respectively are lost compared to the loss of one carbon atom in the instant process. Regarding the principles of "atom economy" introduced by Trost, B.M. (*Science,* 254, **1991,** 1471) a high loss of atoms in chemical reactions is disadvantageously.

EP-A-0 277 002 discloses in Scheme I the oxidation of several steroids with ruthenium dioxide/sodium periodate in an organic solvent leading to the corresponding acid. However, there is no example for said process and the Scheme contains no further details.

In saturated rigid ring structures like steroids, ring carbon atoms have

(a) either two monovalent substituents or one bivalent substituent attached to a carbon atom which is part of one ring and has two ring carbon-carbon bonds, or

(b) one monovalent substituent attached to a carbon atom which is part of two condensed rings and has three ring

carbon-carbon bonds.

Each of the two substituents under (a) is in either an equatorial or axial position relative to the ring and may change between axial/equatorial due to ring flipping. However, the position of the two substituents relative to the ring and each other remain fixed. In structural formulae depicting such ring structures, accordingly to the common numbering of steroid systems, i.e.

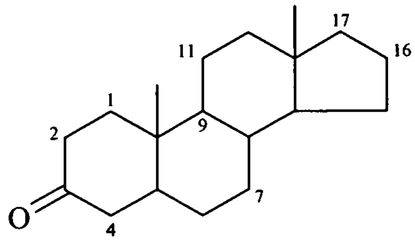

a substituent $R^{n-1}$, wherein n denotes the number of the respective carbon atom $C^n$ which is "below" the other substituent will be identified as being in the α-position (e.g. $R^{1-1}$ or α-$R^1$) and its bond to the carbon atom $C^n$ is represented by a broken, dotted or dashed line. Accordingly, the substituent $R^{n-2}$ attached "above" the other ($R^{n-1}$) is identified as being in the β-position and its bond to the carbon atom $C^n$ is represented by a heavy or bold line or a solid wedge.

Herein, the symbol $Q^n$ in structural formulae represents either one bivalent substituent or two monovalent substituents $R^n$ (i.e. $R^{n-1}$ or $R^{n-2}$).

Where, according to (b), only one monovalent substituent is attached to the ring skeleton (e.g. $R^9$ attached to $C^9$), its relative position is described to be below (i.e. $R^{9-1}$) or above (i.e. $R^{9-2}$) of the ring skeleton.

$R^1$ and $R^2$ are independently selected from the group consisting of hydrogen, F, Cl, Br, I, $C_{1-6}$alkyl and $C_{1-6}$-alkoxy, wherein each alkyl and/or alkoxy moiety is optionally substituted with one or more halogen atoms.

$R^4$ is selected from the group consisting of hydrogen, (*N,N*-di-$C_{1-6}$-alkylamino)methyl, *2-(N,N* di-$C_{1-6}$-alkylamino)ethyl, $C_{1-6}$-alkyl, $C_{1-6}$-alkoxy, phenyl and benzyl, wherein each alkyl and/or alkoxy moiety is optionally substituted with one or more halogen atoms, and wherein each phenyl and/or benzyl moiety is optionally substituted with one or more $NO_2$, F, Cl, Br, I, $C_{1-3}$-alkyl, $C_{1-3}$-alkoxy, dimethylamino or diethylamino groups.

$Q^7$ represents a carbonyl oxygen atom or is $R^{7-1}$ and $R^{7-2}$, wherein one of $R^{7-1}$ and $R^{7-2}$ is hydrogen and the other is selected from the group consisting of hydrogen, F, Cl, Br, I, $C_{1-6}$-alkyl and $C_{1-6}$alkoxy, wherein each alkyl and/or alkoxy moiety is optionally substituted with one or more halogen atoms.

$R^9$ represents hydrogen or F.

$Q^{11}$ represents a carbonyl oxygen atom or is $R^{11-1}$ and $R^{11-2}$, wherein one of $R^{11-1}$ and $R^{11-2}$ is hydrogen and the other is selected from the group consisting of hydrogen, cyano, cyano-$C_{1-3}$-alkyl, acetoxy, COOH and $COO^-M^+$, wherein $M^+$ is $Na^+$, $K^+$ or $NH^+_4$.

$R^{16}$ is selected from the group consisting of hydrogen, cyano, $C_{1-3}$-alkyl, cyano-$C_{1-3}$-alkyl, acetoxy, COOH and $COO^-M^+$, wherein $M^+$ is $Na^+$, $K^+$ or $NH^+_4$.

COOR represents COOH or $COO^-M^+$, wherein $M^+$ is $Na^+$, $K^+$ or $NH^+_4$.

Here and hereinbelow the term "$C_{1-n}$-alkyl" represents a linear or branched alkyl group having 1 to n carbon atoms. $C_{1-10}$-alkyl is for example methyl, ethyl, propyl, isopropyl, butyl, isobutyl, *sec*-butyl, *tert*-butyl, pentyl, 1,4-dimethyl-pentyl, hexyl, heptyl, 1,5-dimethyl-hexyl, decyl or 4-ethyl-1,5-dimethylhexyl.

Here and hereinbelow the term "$C_{1-6}$-alkoxy" represents a linear or branched alkoxy group having 1 to 6 carbon atoms, for example methoxy, ethoxy, propoxy, isopropoxy, butoxy, isobutoxy, *sec*-butoxy, pentyloxy or hexyloxy.

Here and hereinbelow the term "cyano-$C_{1-6}$-alkyl" represents a linear alkyl group having 1 to 6 carbon atoms with to a terminal cyano group, for example cyanomethyl, cyanoethyl, cyanopropyl, cyanobutyl, cyanopentyl or cyanohexyl.

Here and hereinbelow the term "$C_{3-18}$-alkanoic acid" represents a linear or branched alkanoic acid having 3 to 18 carbon atoms, for example propionic acid, butyric acid, octanoic acid, nonanoic acid, decanoic acid, undecanoic acid, dodecanoic acid or octadecanoic acid.

Here and hereinbelow the term "$C_{8-18}$-alkanesulfonic acid" represents a linear or branched alkanesulfonic acid having 8 to 18 carbon atoms, for example octanesulfonic acid, dode-canesulfonic acid.

Here and hereinbelow the term "$C_{1-6}$-alkylamino" represents a linear or branched alkylamino group having 1 to 6 carbon atoms, for example methylamino, ethylamino, propylamino, butylamino, pentylamino or hexylamino.

Here and hereinbelow the term "$C_{1-6}$-alkylamine" represents a linear or branched alkylamine having 1 to 6 carbon atoms, for example methylamine, ethylamine, propylamine, butylamine, pentylamine or hexylamine.

Here and hereinbelow the term "$C_{1-4}$-alcohol" represents a linear or branched alcohol having 1 to 4 carbon atoms, i.e. methanol, ethanol, propanol, isopropanol, n-butanol, *sec*-butanol and *tert*-butanol.

The technical problem to be solved was to provide an industrially applicable process for the preparation of steroidal seco acids wherein the oxidative cleavage of the steroidal A-ring can be separated from the formation of the carboxyl group attached to $C^{17}$ of the steroidal skeleton. Furthermore the use of expensive agents like $NaIO_4$ should be avoided. A further object of the invention was to establish an economically advantageous reaction sequence for the preparation of 4-azasteroid intermediates of formula I, requiring a minimum of work-up and isolation steps for compounds of formula IV.

The problems above could be solved according to the process of claim 1.

To avoid the drawbacks in the known processes, a process was established which allows formation of the carboxyl group attached to $C^{17}$ of the steroidal skeleton and purification of the compound of formula III prior to the cleavage of the steroidal A-ring. Since isolated oxidation of the ene-oxo side chain of the compounds disclosed in WO 90/15045 is not possible without infliction of the A-ring, the carboxyl group attached to $C^{17}$ had to be introduced by a complete different reaction regime and different starting compounds. By subjecting to a haloform reaction compounds of formula II, containing an acetyl group attached to $C^{17}$, can be converted to compounds of formula III easily and in high yield. The haloform reaction is completely indifferent to the ene-oxo fragment in the steroidal A-ring and compounds of formula III can be purified to comply with requirements for steroid drugs. The decoupling of both oxidation steps allows usage of starting compounds of poor quality.

Provided is a process for the preparation of 4-aza-steroidal acids of the formula

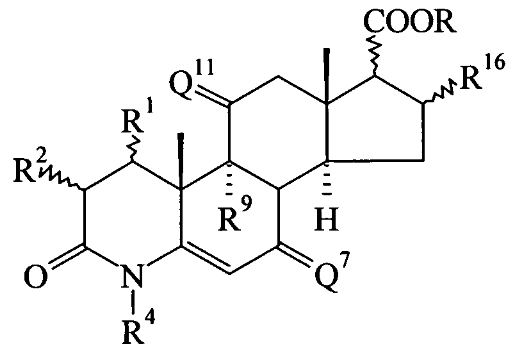

I,

wherein
$R^1$, $R^2$, $R^4$, $Q^7$, $R^9$, $Q^{11}$, $R^{16}$ and COOR are as defined above,
comprising three steps of

(i) converting a compound of formula

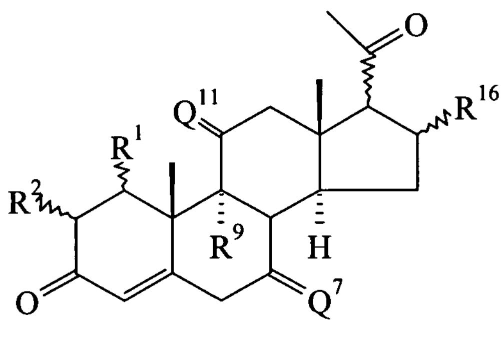

II,

wherein $R^1$, $R^2$, $Q^7$, $R^9$, $Q^{11}$ and $R^{16}$ are as defined above, by a haloform reaction, optionally in the presence of a polar organic additive, into a compound of formula

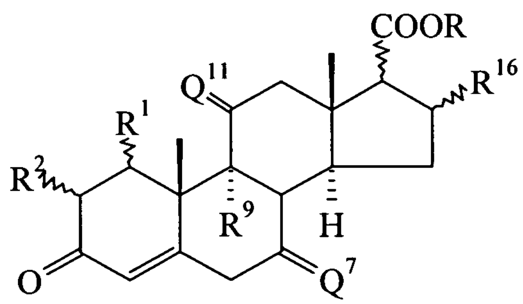

III,

wherein $R^1$, $R^2$, $Q^7$, $R^9$, $Q^{11}$, $R^{16}$ and COOR are as defined above, which is
(ii) reacted by ozonolysis and oxidative work-up procedure into a compound of formula

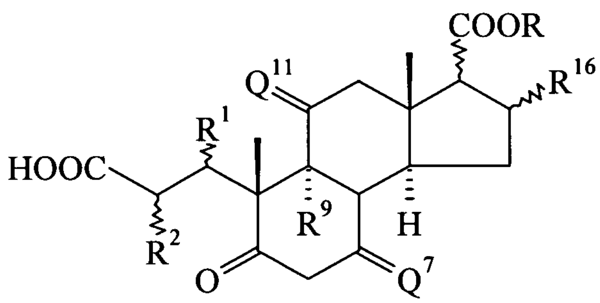

IV,

wherein $R^1$, $R^2$, $Q^7$, $R^9$, $Q^{11}$, $R^{16}$ and COOR are as defined above, which is
(iii) cyclized with an nitrogen compound of the formula $H_2NR^4$, wherein $R^4$ is as defined above, into the compound of formula I.

In a preferred embodiment the haloform reaction of step (i) is a bromoform reaction.

Preferably said haloform reaction is carried out in aqueous solution, optionally in the presence of a polar organic additive. Such additive, being inert under the reaction conditions of the haloform reaction, may act as a solubilizer of the starting compound. In a preferred embodiment the polar organic additive is selected from the group consisting of *tert*-butylalcohol, acetonitrile and propionitrile.

Preferably step (i) is carried out in the presence of a hydroxide ion releasing base. Such a base can be an alkali and/or earth alkali hydroxide or a bicarbonate or carbonate, which is able to release hydroxide ions by deprotonating water present in the reaction mixture. Particularly preferred the base is selected from the group consisting of LiOH, NaOH, KOH, $Ba(OH)_2$, $Mg(OH)_2$, $Ca(OH)_2$, $NaHCO_3$, $KHCO_3$, $Na_2CO_3$, $K_2CO_3$ and mixtures thereof, more preferably NaOH, KOH, $NaHCO_3$ and mixtures thereof.

Optionally, step (i) is followed by a purification step. Due to the higher polarity and the possibility of form salt formation of compounds of formula III may be separated from compounds of formula II by liquid-liquid extraction. Another appropriate purification method is recrystallization of the product in a $C_{1-4}$-alcohol/water mixture. Preferably a methanol/water mixture at a temperature of 30 to 80 °C is applied.

Advantageously the ozonolysis is carried out by passing ozone through a solution of a compound of formula III in an inert solvent. Preferably the solvent of step (ii) is selected from the group consisting of $H_2O$, $C_{1-4}$-alcohols, acetonitrile, propionitrile, methylene chloride and chloroform.

In a preferred embodiment step (ii) is carried out at a temperature below -10 °C, more preferably at or below -15 °C.

The ozonolysis is applied until a sufficient conversion is reached, then oxygen is passed through the reaction mixture to remove at least the major amount of excess ozone. For the oxidative work-up procedure a hydroxide ion releasing base is added to the reaction mixture. A suitable hydroxide ion releasing base is alkali and/or earth alkali hydroxide or a bicarbonate or carbonate, which is able to release hydroxide ions by deprotonating water present in the reaction mixture. Particularly preferred the base is selected from the group consisting of LiOH, NaOH, KOH, $Ba(OH)_2$, $Mg(OH)_2$, $Ca(OH)_2$, $NaHCO_3$, $KHCO_3$, $Na_2CO_3$, $K_2CO_3$ and mixtures thereof, more preferably NaOH, KOH, $NaHCO_3$ and mixtures thereof. Completion of the oxidative work-up procedure can be followed for example by redox titration or polarography.

When the oxidative work-up procedure is finished for removal of residual ozone and possibly formed peroxides,

the reaction mixture is then treated with a reducing agent. In a preferred embodiment the reducing agent is selected from the group consisting of $NaHSO_3$, $KHSO_3$, $SO_2$, tri-$C_{1-3}$-alkyl-phosphines, tri-$C_{1-3}$-alkyl-phosphites, triphenylphosphine and mixtures thereof, preferably $NaHSO_3$ or $SO_2$.

Particularly preferred the reaction mixture is maintained at a temperature between 20 and 100 °C for at least 5 minutes after addition of the reducing agent. Complete removal of peroxides and ozone is time and temperature dependent. At 60 °C a reduction time of below 1 hour is often sufficient, at 100 °C some minutes will be sufficient.

After the ozonolyis and oxidative work-up procedure has finished the salts of the acids of formula III remain in the basic aqueous solution. To obtain the protonated compounds of formula III preferably a liquid-liquid extraction is applied. More preferably before the extraction the pH of the aqueous phase is adjusted to below pH 7, more preferably between pH 3 and 6.

In another particularly preferred embodiment after removal of excess oxidants and adjusting the pH to below 7, Celite® is added to the aqueous solution and the mixture is filtered to remove sticky compounds and to enhance the product quality.

In a further particularly preferred embodiment the liquid-liquid extraction is carried out using *tert*-butyl methyl ether (TBME), $CHCl_3$, $CH_2Cl_2$, 1,1,1-trichloroethane, 1,2-dichloropropane, methyl acetate or ethyl acetate. Preferably the extraction of the aqueous mixture is carried out with ethyl acetate at an pH between 4.5 and 5.5.

In a preferred process using $CH_2Cl_2$ for the extraction, optionally the product is precipitated from $CH_2Cl_2$ by change of the polarity of the solvent, by adding an apolar solvent like methyl cyclohexane.

Preferably, the cyclization reaction of step (iii) is carried out between 80 and 150 °C, particularly preferred between 110 and 140 °C.

In a further preferred embodiment, the cyclization reaction is carried out in the presence of a non-oxidizing proton acid and/or a polar organic additive to facilitate solvatisation. Preferably the non-oxidizing proton acid is an organic or inorganic acid selected from the group consisting of acetic acid, propionic acid, butyric acid, isobutyric acid, benzoic acid, HCl, HBr, HI and mixtures thereof. Particularly preferred, the proton acid is acetic or propionic acid.

Preferably the polar organic additive is selected from the group consisting of *tert*-butyl methyl ether, $C_{1-4}$-alkyl alcohols, ethylene glycol, acetonitrile and dimethyl sulfoxide.

In a preferred embodiment $R^4$ in the nitrogen compound of formula $H_2NR^4$ is hydrogen or $C_{1-6}$-alkyl, optionally substituted with one or more halogen atoms. In a further preferred embodiment soluble salts of the nitrogen compounds of formula $H_2NR^4$ are used, for example $NH_4Cl$, $NH_4Br$, ammonium acetate, ammonium propionate, ammonium butyrate, or hydrochlorides or hydrobromides of $C_{1-6}$-alkylamines. In a further preferred embodiment the nitrogen compound of the formula $H_2NR^4$ or a salt thereof can be used as a solution in the presence of a non-oxidizing proton acid and/or a polar additive, as defined above.

Particularly preferred the nitrogen compound is selected from the group consisting of $C_{1-6}$-alkylamines, soluble salts thereof, $NH_3$, $NH_4Cl$ $NH_4Br$ and $NH_4OAc$, preferably $NH_3$ and $NH_4OAc$.

In a particularly preferred embodiment 3-oxo-4-aza-androst-5-ene-17β-carboxylic acid of formula

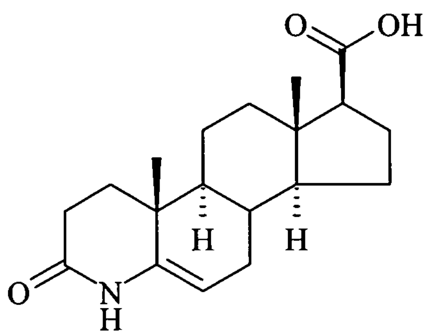

Ia

is prepared, wherein

(i) progesterone of formula

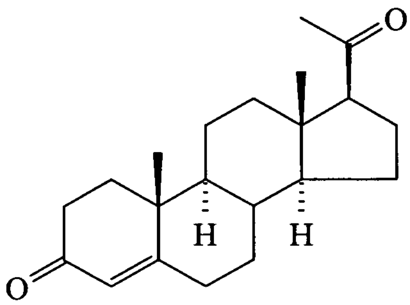

IIa

or a salt thereof, is reacted by a bromoform reaction, optionally in the presence of a polar organic additive, into progesteronic acid of formula

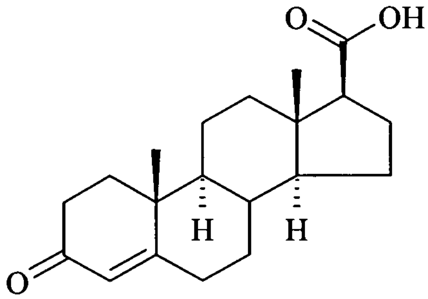

IIIa

or a salt thereof, which is,

(ii) reacted by ozonolysis and oxidative work-up into 17β-carboxy-5-oxo-A-nor-3,5-seco-androstan-3-oic acid of formula

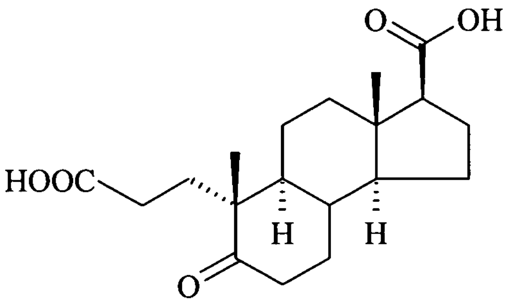

IVa

or a salt thereof, which is

(iii) cyclized by reaction with $NH_3$ or a soluble salt thereof, preferably with $NH_3$ in ethylene glycol or $NH_4OAc$ in AcOH, into compound Ia or a salt thereof.

In a preferred embodiment the bromoform reaction of compound IIa in step (i) is carried out in the presence of *tert*-butanol.

The ozonolysis of compound IIIa in step (ii) is preferably carried out in methanol below - 10 °C. In a further preferred embodiment the oxidative work-up is carried out at a pH >9. After oxidative work-up is finished, peroxides are preferably removed using $NaHSO_3$ as reducing agent.

In a preferred embodiment cyclization of compound IVa in step (iii) is carried out with $NH_4OAc$ at a temperature of 110 to 140 °C. Optionally step (iii) is carried out in the presence of a non-oxidizing proton acid, preferably in the presence of acetic acid.

The present invention is illustrated by the following non-limiting examples.

**Examples:**

**Example 1:** 3-Oxo-4-androst-4-ene-17β-carboxylic acid (IIIa of formula III with $R^1 = R^2 = R^{7-1} = R^{7-2} = R^9 = R^{11-1} = R^{11-2} = R^{16}$ = H, COOR = COOH)

A three-neck 1 L reactor equipped with a mechanical stirrer and thermometer is charged, under nitrogen atmosphere, with 30% NaOH (379 g, 2840 mmol, 9.46 eq) and distilled water (459 mL). The NaOH the solution was cooled to 0 to 5 °C, and bromine (214.8 g, 1344 mmol, 4.48 eq) was introduced over a period of at least 60 min to form an orange solution of sodium hypobromite. The solution was then stirred an additional 30 min at 0 °C prior to use. A three-neck 3 L reactor equipped with a mechanical stirrer, thermometer and distillation head is charged, under nitrogen atmosphere, with progesterone (IIa, 94.5 g, 300 mmol, 1.0 eq), *tert*-butanol (910 mL) and stirred at 25 °C. A solution of 30% NaOH (197 g, 1477 mmol, 4.92 eq) and distilled water (474 mL) was then added over a period of 70 min, while the temperature was held at 25 °C. The yellow solution was cooled to 0 °C before being treated with NaOBr solution over a period of 60 min. The heterogeneous mixture was then stirred for 3 h at 0 °C.

After completion of the reaction a solution of sodium sulfite (37.8 g, 364 mmol, 1.21 eq) in water (147 mL) was added within 15 min to the cold reaction mixture (slightly exothermic). The solution was aged overnight (15 h) at 20 °C. *tert*-Butylalcohol was then removed by distillation at 23 to 37 °C and a pressure of 90 to 65 mbar. 807 g of alcohol were removed from the reaction mixture in this way. Distilled water (1200 mL) was then added to the rose coloured reaction mixture, followed by toluene (300 mL), and the mixture warmed to 35 °C. After 15 min the stirring was stopped and the phases allowed to separate. The separated aqueous phase (2780 g, pH 14) was then acidified to pH 3 over a 90 minute period by the addition of 37% HCl (244 g, 2476 mmol, 8.25 eq), and the resulting solution was stirred at room temperature for an additional 60 min before being filtered. The filter cake was washed with distilled water (2x180 mL) and the wet product (217 g) dried under vacuum (45 °C, approx. 20 mbar). 85.5 g of IIIa (69%, HPLC 77% w/w, 94% area) of 3-oxo-4-androst-4-ene-17β-carboxylic acid (etiocholenic acid) was isolated.

**Example 2:** Purification of 3-oxo-4-androst-4-ene-17β-carboxylic acid (IIIa of formula III with $R^1 = R^2 = R^{7-1} = R^{7-2} = R^9 = R^{11-1} = R^{11-2} = R^{16}$ = H, COOR = COOH)

A three-neck 2 L reactor equipped with a mechanical stirrer, thermometer and distillation head is charged, under nitrogen atmosphere, with crude etiocholenic acid (170 g, HPLC 77% w/w), methanol (1360 mL), and distilled water (340 mL) stirred and then warmed to reflux (65 °C). The suspension was stirred for 2 h. and then cooled to 0 °C over a 75 min period. The resulting suspension was filtrated and the filter cake was washed with cooled (60:40) methanol / water solution (2×100 mL). After drying under vacuum (45 °C, approx. 20 mbar), 110 g (84%, HPLC 99% w/w, 99% area) of pure etiocholenic acid (IIIa) were obtained.

**Example 3:** 17β-Carboxy-5-oxo-A-nor-3,5-secoandrostan-3-oic acid (IVa of formula IV with $R^1 = R^2 = R^{7-1} = R^{7-2} = R^9 = R^{11-1} = R^{11-2} = R^{16}$ = H, COOR = COOH)

Etiocholenic acid (IIIa, 79.1 g, 250 mmol) was placed in the ozonolysis vessel under nitrogen and taken up in methanol (750 mL). The mixture was cooled to -15 °C (internal temperature), and treated with ozone for about 4 hours. after reaction were completed the flow of ozone was stopped and $O_2$ was bubbled through the mixture for 15 min. The reaction mixture was then placed under $N_2$, and water (425 mL) was then added at the same temperature, resulting the precipitation of a white solid. Over the following 2 h the mixture was allowed to warm from -15 to 40 °C. For oxidative work-up, the reaction mixture is taken up to pH 11 by addition of 30% sodium hydroxide (93 mL). The reaction mixture was concentrated under vacuum (40 °C, approx. 150 mbar) (492 g, distillate) and the remaining yellow solution (713 g) extracted, at room temperature, with *tert*-butyl methyl ether (500 mL). The organic phase was separated and treated with 40% $NaHSO_3$ (1 ×25 mL) to eliminate peroxides, then concentrated on the rotorvapor (35 °C and 30 mbar) to give a light yellow product (100 g). The residual aqueous solution was then over a period of 30 min adjusted to pH 3 by treating with 35% HCl (65 mL) at room temperature, before being extracted with tert-butyl methyl ether (600 mL). Separation of the organic solution, followed by washing with 40% $NaHSO_3$ (1 ×25 mL) to eliminate peroxides and then with water (1 ×25 mL). By concentration on a rotorvapor, (35 °C, 20 mbar) and drying (16 h) under vacuum 92.7 g (HPLC 72% w/w, 73% area) fine white powder of 17β-carboxy-5-oxo-A-nor-3,5-secoandrostan-3-oic acid (IVa) were obtained.

**Example 4:** 3-Oxo-4-aza-androst-5-ene-17β-carboxylic acid (Ia of formula I with $R^1 = R^2 = R^4 = R^{7-1} = R^{7-2} = R^9 = R^{11-1} = R^{11-2} = R^{16}$ = H, COOR = COOH)

A three-neck 1 mL reactor equipped with a mechanical stirrer, a thermometer and a distillation head is charged,

under nitrogen atmosphere, with a *tert*-butyl methyl ether solution of 17β-carboxy-5-oxo-A-nor-3,5-secoandrostan-3-oic acid (IVa, 700 mL). The solvent is distilled under vacuum (100 mbar / bath approx. 60 °C) to 1/3 of the initial volume. Acetic acid (760 mL) is then added and distillation continued until acetic acid starts to distil. Ammonium acetate (132.5 g, 1700 mmol, approx. 7 eq) is added and the reaction mixture is heated to reflux (approx. 120 °C) within 1 h (bath temp.: approx. 140 °C). After 1h and 2 h, the conversion is checked by TLC. The reaction mixture is then concentrated (40% of acetic acid distilled off). The reaction mixture (yellow-orange suspension) is cooled down within 1h to approx. 60 °C. Water (830 mL) is then slowly added (approx. 1 h) to the reaction mixture at approx. 60 °C and the suspension slowly (1h) cooled to 20 °C. After 0.5 h (20 °C), the suspension is filtered and the filter cake was washed with water (2x100 mL) and a solution of water/ethanol (1:1; v:v) (2x100 mL). Drying at 80 °C (approx. 20 mbar / approx. 16 h) afforded 68.9 g (83% overall yield, assay corrected, from etiocholenic acid) of light tan product of 3-oxo-4-aza-androst-5-ene-17β-carboxylic acid (Ia) (HPLC 99% area, 95.4% w/w).

**Example 5:** 3-Oxo-4-aza-androst-5-ene-17β-carboxylic acid (Ia of formula I with $R^1 = R^2 = R^4 = R^{7-1} = R^{7-2} = R^9 = R^{11-} = R^{11-2} = R^{16}$ = H, COOR = COOH)

To the conc. solution of 17β-carboxy-5-oxo-A-nor-3,5-secoandrostan-3-oic acid (IVa) (450 g, 1.33 mol), 2100 mL of acetic acid are added and ethyl acetate is distilled off. The cooled mixture is then transferred, under nitrogen atmosphere, to a three-neck 10 L reactor, equipped with a mechanical stirrer, a thermometer and a distillation head, and than charged with ammonium acetate (560 g, 7.2 mol). The reaction mixture is heated to reflux within 1 h and than cooled down within 0.5 h to 60 °C. Water (3360 mL) is then slowly added and the suspension slowly cooled to 20 °C. After 0.5 h, the suspension is filtered and the filter cake was washed with water (2x840 mL) and a mixture of water/ethanol [v:v, 1:1] (3x840 mL). Drying at 50°C (approx. 20 mbar / approx. 16 h) affords 355 g (74.5% overall yield) of white to light tan of 3-oxo-4-aza-androst-5-ene-17β-carboxylic acid (Ia) at a purity of about 100%.

**Comparative example 1:** 17β-Carboxy-5-oxo-A-nor-3,5-secoandrostan-3-oic acid (Formula I, $R^1 = R^2 = R^{7-1} = R^{7-2} = R^9 = R^{11-1} = R^{11-2} = R^{16}$ = H, COOR = COOH)

A three-neck 1 L reactor, equipped with a mechanical stirrer, a thermometer, a device for pH-controlled NaOH addition and a bubble counter, is purged with nitrogen and then charged with 200 mL of deionized water, 9.7 g of $NaHCO_3$ (115.5 mmol), 16.2 g of etiocholenic acid (50 mmol) and 58 mg of $RuCl_3 \cdot H_2O$ (0.37 mmol) and 16 mL of acetonitrile. The slurry is cooled down to 10 °C under vigorous stirring (approx. 1000 rpm). The solution is first adjusted to pH 8.0 by addition of NaOH (30%). A solution of 105.9 g of OXONE® (345 mmol, monopersulfate compound $2KHSO_5 \cdot KHSO_4 \cdot K_2SO_4$, from Aldrich) in 425 mL of deionized water is added to the stirred suspension within 3.5 h, while the mixture is maintained between pH 8.0 and 8.4 by addition of 30% NaOH solution. The reaction mixture is aged for 1.5 h. After complete conversion of etiocholenic acid, 4.3 g of $NaHSO_3$ (40 mmol) is added. The reaction mixture is warmed up to 60 °C for 1 h (colour changes from brown to light green) and then cooled to 20 °C. $CH_2Cl_2$ (254 mL) is added. The pH is adjusted to 1.5 by the addition of conc. HCl. After phase separation the water phase is extracted with 80 mL $CH_2Cl_2$. The combined organic layers are then filtered, dried over $Na_2SO_4$. Residual water is removed from the moist product at 55 °C under vacuum. Yield is approx. 90%, by HPLC, purity is approx. 60%.

## Claims

1. A process for the preparation of 4-azasteroids of the formula

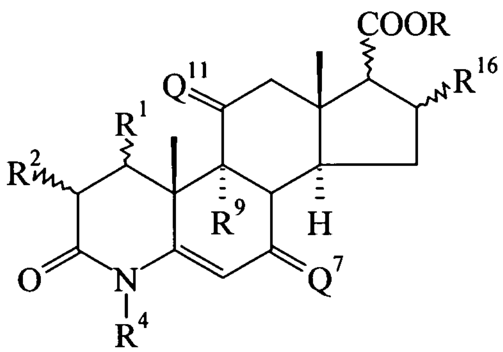

I,

wherein
$R^1$ and $R^2$ are independently selected from the group consisting of hydrogen, F, Cl, Br, I, $C_{1-6}$-alkyl and $C_{1-6}$-alkoxy, wherein each alkyl and/or alkoxy moiety is optionally substituted with one or more halogen atoms, and
$R^4$ is selected from the group consisting of hydrogen, (*N,N*-di-$C_{1-6}$-alkylamino)-methyl, 2-(*N,N*-di-$C_{1-6}$-alkylamino)ethyl, $C_{1-6}$-alkyl, $C_{1-6}$-alkoxy, phenyl and benzyl, wherein each alkyl and/or alkoxy moiety is optionally substituted with one or more halogen atoms, and wherein each phenyl and/or benzyl moiety is optionally substituted with one or more $NO_2$, F, Cl, Br, I, $C_{1-3}$-alkyl, $C_{1-3}$-alkoxy, dimethylamino or diethylamino groups, and
$Q^7$ represents a carbonyl oxygen atom or is $R^{7-1}$ and $R^{7-2}$, wherein one of $R^{7-1}$ and $R^{7-2}$ is hydrogen and the other is selected from the group consisting of hydrogen, F, Cl, Br, I, $C_{1-6}$-alkyl and $C_{1-6}$-alkoxy, wherein each alkyl and/or alkoxy moiety is optionally substituted with one or more halogen atoms, and
$R^9$ represents hydrogen or F, and
$Q^{11}$ represents a carbonyl oxygen atom or is $R^{11-1}$ and $R^{11-2}$, wherein one of $R^{11-1}$ and $R^{11-2}$ is hydrogen and the other is selected from the group consisting of hydrogen, cyano, cyano-$C_{1-3}$-alkyl, acetoxy, COOH and $COO^-M^+$, wherein $M^+$ is $Na^+$, $K^+$ or $NH^+_4$ , and
$R^{16}$ is selected from the group consisting of hydrogen, cyano, $C_{1-3}$-alkyl, cyano-$C_{1-3}$-alkyl, acetoxy, COOH and $COO^-M^+$, wherein $M^+$ is $Na^+$, $K^+$ or $NH^+_4$, and COOR is COOH or $COO^-M^+$, wherein $M^+$ is $Na^+$, $K^+$ or $NH^+_4$,
comprising three steps of

(i) converting a compound of formula

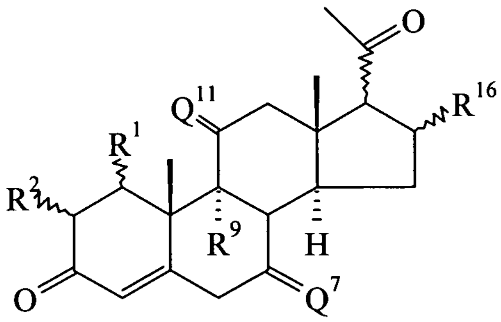

II,

wherein $R^1$, $R^2$, $Q^7$, $R^9$, $Q^{11}$ and $R^{16}$ are as defined above, by a haloform reaction, optionally in the presence of a polar organic additive, into a compound of formula

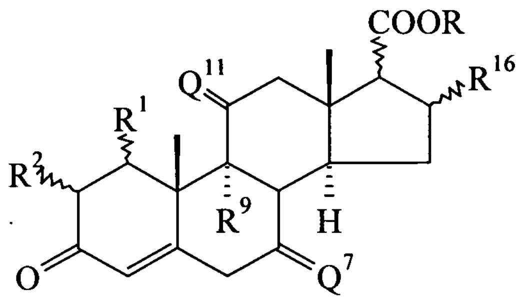

III,

wherein $R^1$, $R^2$, $Q^7$, $R^9$, $Q^{11}$, $R^{16}$ and COOR are as defined above, which is
(ii) reacted by ozonolysis and oxidative work-up procedure into a compound of formula

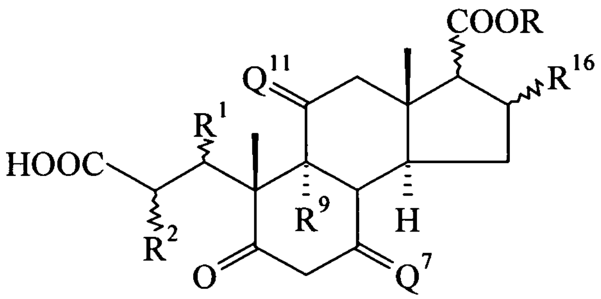

IV,

wherein $R^1$, $R^2$, $Q^7$, $R^9$, $Q^{11}$, $R^{16}$ and COOR are as defined above, which is
(iii) cyclized with a nitrogen compound of the formula $H_2NR^4$, wherein $R^4$ is as defined above, into the compound of formula I, wherein $R^1$, $R^2$, $R^4$, $Q^7$, $R^9$, $Q^{11}$, $R^{16}$ and COOR are as defined above.

**2.** The process of claim 1, wherein the haloform reaction is a bromoform reaction.

**3.** The process of claims 1 or 2, wherein the polar organic additive is selected from the group consisting of *tert*-butylalcohol, acetonitrile and propionitrile.

**4.** The process of any of claims 1 to 3, wherein step (ii) is carried out in a solvent selected from the group consisting $H_2O$, $C_{1-4}$-alcohol, acetonitrile, propionitrile, methylene chloride and chloroform.

**5.** The process of any of claims 1 to 4, wherein at the end of step (ii) after oxidative work-up procedure a reducing agent is added to the reaction mixture to remove peroxides.

**6.** The process of any of claims 1 to 5, wherein in the nitrogen compound of the formula $H_2NR^4$ in step (iii) is selected from the group consisting of $C_{1-6}$-alkylamines, soluble salts thereof, $NH_3$, $NH_4Cl$, $NH_4Br$ and $NH_4OAc$.

**7.** The process of any of claims 1 to 6, wherein step (iii) is carried out at a temperature of 80 to 150 °C, preferably of 110 to 140 °C.

**8.** The process of claims 1 to 7, wherein step (iii) is carried out in the presence of a non-oxidizing proton acid selected from the group consisting of acetic acid, propionic acid, butyric acid, isobutyric acid, benzoic acid, HCl, HBr and HI.

**9.** The process of any of claims 1 to 8, wherein step (iii) is carried out in the presence of a polar organic additive selected from the group consisting of *tert*-butyl methyl ether, $C_{1-4}$-alkyl alcohols, ethylene glycol, acetonitrile and dimethyl sulfoxide.

**10.** The process of any of claims 1 to 9, for the preparation of 3-oxo-4-aza-androst-5-ene-17β-carboxylic acid of formula I with $R^1 = R^2 = R^4 = R^{7-1} = R^{7-2} = R^9 = R^{11-1} = R^{11-2} = R^{16} = H$ and COOR = COOH (Ia) wherein step (ii) is a bromoform reaction, and wherein step (iii) is carried out by reacting 17β-carboxy-5-oxo-A-nor-3,5-seco-androstan-3-oic acid of formula IVa or a salt thereof with $NH_3$ or a soluble ammonium salt, preferably with $NH_3$ in ethylene glycol or $NH_4OAc$ in AcOH, into compound Ia or a salt thereof.

European Patent Office

# EUROPEAN SEARCH REPORT

Application Number
EP 04 01 5746

| Category | Citation of document with indication, where appropriate, of relevant passages | Relevant to claim | CLASSIFICATION OF THE APPLICATION (Int.Cl.7) |
|---|---|---|---|
| A | WO 95/12398 A (MERCK & CO INC ; BAKSHI RAMAN K (US); PATEL GOOL F (US); RASMUSSON GAR) 11 May 1995 (1995-05-11)<br>* page 23 *<br>----- | 1-10 | C07J73/00 |
| A | RASMUSSON G H ET AL: "AZASTEROIDS AS INHIBITORS OF RAT PROSTATIC 5ALPHA-REDUCTASE"<br>JOURNAL OF MEDICINAL CHEMISTRY, AMERICAN CHEMICAL SOCIETY. WASHINGTON, US,<br>vol. 27, no. 12,<br>1 December 1984 (1984-12-01), pages 1690-1701, XP002043194<br>ISSN: 0022-2623<br>* Scheme I *<br>----- | 1-10 | |
| A | RASMUSSON G H ET AL: "AZASTEROIDS: STRUCTURE-ACTIVITY RELATIONSHIPS FOR INHIBITION OF 5ALPHA-REDUCTASE AND OF ANDROGEN RECEPTOR BINDING"<br>JOURNAL OF MEDICINAL CHEMISTRY, AMERICAN CHEMICAL SOCIETY. WASHINGTON, US,<br>vol. 29, no. 11,<br>1 November 1986 (1986-11-01), pages 2298-2315, XP000568779<br>ISSN: 0022-2623<br>*Scheme 1*<br>----- | 1-10 | TECHNICAL FIELDS SEARCHED (Int.Cl.7)<br>C07J |
| A | BRATOEFF E ET AL: "STEROIDAL ANTIANDROGENS AND 5ALPHA-REDUCTASE INHIBITORS"<br>CURRENT MEDICINAL CHEMISTRY, BENTHAM SCIENCE PUBLISHERS BV, BE,<br>vol. 6, no. 12, December 1999 (1999-12), pages 1107-1123, XP000874719<br>ISSN: 0929-8673<br>* the whole document *<br>-----<br>-/-- | 1-10 | |

The present search report has been drawn up for all claims

| Place of search | Date of completion of the search | Examiner |
|---|---|---|
| Munich | 17 November 2004 | Baston, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

1

EPO FORM 1503 03.82 (P04C01)

European Patent Office

# EUROPEAN SEARCH REPORT

Application Number
EP 04 01 5746

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | ISHIBASHI, K. ET AL.: "Synthesis and testosterone 5 alpha-reductase inhibitory activity of 11-substituted 4-aza-5alpha-androstane compounds" EUROPEAN JOURNAL OF MEDICINAL CHEMISTRY, vol. 31, 1996, XP002306058 * Scheme 1* ----- | 1-10 | |
| | | | TECHNICAL FIELDS SEARCHED (Int.Cl.7) |

1

The present search report has been drawn up for all claims

| Place of search | Date of completion of the search | Examiner |
|---|---|---|
| Munich | 17 November 2004 | Baston, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 04 01 5746

This annex lists the patent family members relating to the patent documents cited in the above-mentioned European search report.
The members are as contained in the European Patent Office EDP file on
The European Patent Office is in no way liable for these particulars which are merely given for the purpose of information.

17-11-2004

| Patent document cited in search report | Publication date | Patent family member(s) | Publication date |
|---|---|---|---|
| WO 9512398 A | 11-05-1995 | AT 178795 T | 15-04-1999 |
| | | AU 698696 B2 | 05-11-1998 |
| | | AU 8130194 A | 23-05-1995 |
| | | CA 2174234 A1 | 11-05-1995 |
| | | DE 69417925 D1 | 20-05-1999 |
| | | DE 69417925 T2 | 25-11-1999 |
| | | EP 0748221 A1 | 18-12-1996 |
| | | JP 9504553 T | 06-05-1997 |
| | | WO 9512398 A1 | 11-05-1995 |

EPO FORM P0459

For more details about this annex : see Official Journal of the European Patent Office, No. 12/82